# EUROPEAN PATENT SPECIFICATION

(11) **EP 1 011 613 B1**
(45) Date of publication and mention of the grant of the patent: **15.10.2003**
(21) Application number: 98902171.2
(22) Date of filing: 22.01.1998
(51) Int. Cl.: A61K 7/06

(54) **OXYGEN-OZONE METHOD FOR PROMOTING HAIR GROWTH**
VERFAHREN ZUR FÖRDERUNG DES HAARWACHSTUMS MIT VERWENDUNG VON SAUERSTOFF UND OZON
TECHNIQUE DE STIMULATION DE LA POUSSE DES CHEVEUX REPOSANT SUR UNE UTILISATION D'OXYGENE-OZONE

(30) Priority: 28.01.1997 IT PI970006
(43) Date of publication of application: 28.06.2000
(73) Proprietor: Gamba, William, 17021 Alassio (IT)
(72) Inventor: Gamba, William, 17021 Alassio (IT)
(86) International application number: IT9800009
(87) International publication number: WO98032414

(56) References cited:
- US-A- 3 794 028
- PATENT ABSTRACTS OF JAPAN vol. 014, no. 456 (C-0765), 2 October 1990 & JP 02 180808 A (LION CORP), 13 July 1990,
- PATENT ABSTRACTS OF JAPAN vol. 096, no. 010 & JP 08 150221 A (SERESU KOSUME PLAN KK), 11 June 1996,
- CHEMICAL ABSTRACTS, vol. 112, no. 24, 11 June 1990 Columbus, Ohio, US; abstract no. 223127, "Hair tonics containing ozone" XP002069595 & JP 01 213 216 A (BIOTECH. K.K.)

## Description

### Technical Field

Aesthetical Treatment.

### Background Art

The hereby described invention aims at resolving the problem of hair loss, phenomenon which affects a wide portion of the population (especially the male one: nearly two out of three men develop some form of balding) and which is deemed by a large share of persons an aesthetical problem to solve.

It is common that, as people age, their rate of hair growth slows down. However, it is widespread the phenomen of consistent hairloss, that can be due to many different causes. Some of them are patological or external or diet-related, with effects on hair growth that vary depending on the evolution of the related disease or external event:
- high fevers, severe infections, thyroid diseases;
- childbirth or assumption of birth control pills (for women);
- inadequate proteins or iron in diet;
- assumption of particular drugs or medications (blood thinners; drugs used to treat gout and arthritis, depression, heart problems or high blood pressure; drugs for treating cancer);
- chemotherapy;
- *alopecia areata* (with the advanced forms of *alopecia totalis* and *alopecia universalis*);
- physical or emotional stress.

The most common cause of baldness or hairloss (95% of cases) is however the so-called *androgenetic alopecia*, that is the well-known tendency to balding or thinning, developing in twenty, thirty or fourthy aged persons.

So far, variuos approaches have been conceived to limit or remedy hair loss:
**a)** surgical techniques:
   - self-grafting or transplants: sound bulbs are drawn from the hair-bearing posterior scalp and redistributed in a balding area;
   - flap surgery: a large horseshoe-shape piece of scalp is partially detached from the donor fringe area and the free end is positioned over the bald spot where a corresponding patch of hairless scalp has been removed;
   - scalp reductions: a section of bald scalp is removed and the sides of scalp are lifted and sutured together, thereby reducing the overall surface area of the scalp;
   - scalp expansion and extension: silicone bags are inserted beneath an area of hairy scalp and gradually inflated with a saline water over a six-week period. This causes the hair-bearing skin to stretch, thus increasing the amount of hair-bearing scalp. After removing the bags, expanded hair-bearing skin is lifted and moved to an adjacent bald area where a similar-sized patch of scalp has been excised.

   Even though the use of laser and particular cicatrizing substances has reduced many inconveniences of cicatrization, the surgical method anyway keeps traumatic and painful, still producing undesired effects;
**b)** non-surgical additions of hair bearing devices, that can consist of human hair, synthetic fiber or a combination of both. Those additions are attached in a variety of techniques, either the existing hair or the skin being possible anchor sites;
**c)** medicinal treatments: the most common ones consists of the topical application of vasodilatator substances (such as minoxidil, the "101 regrowth solution", viprostol, diazoxide) which cause the reactivation of the cutaneous blood flow, thus producing a new afflux of oxygen and nutritive material necessary for the regeneration of tissues (in this case, the scalp); or of the assumption of anti-androgens (such as spironolactone, aldactone, cimetidine) or 5-alpha-reductase inhibitors (such as oral finasteride), able to avoid the transformation of testosterone into the dihydrotestoterone form or DHT, held responsible for hair loss.

The topical application of the substances used so far makes the ability of cutaneous absorbment limited and superficial: that is unavoidable, given that, if the vasodilatators should be absorbed in massive manner, undesired systemic effects would derive. For this reason, with regard to hair loss the results obtained until now are not entirely satisfactory.

As for the antiandrogen therapy, is is hard to limit the effects of the androgen blocker to the scalp only, with undesired effects especially for men (e.g. decreased libido, impotence and gynecomastia).

For references, see, *inter alia,* FRIEDEN I.J. - PRICE V.H., *Androgenetic Alopecia,* in

THIERS B.H. - DOBSON R.L. (EDS.), *Pathogenesis of Skin Disease,* New York, 1986, p. 41; AMERICAN ACADEMY OF DERMATOLOGY, *Hair Loss Pamphlet,* Schaumburg, 1987-1993; ORFANOS C.E., *Androgenetic Alopecia: Clinical Aspects and Treatment,* in ORFANOS C.E. - HAPPLE R. (EDS.), *Hair and Hair Disease,* New York, 1990, p. 485; BRODLAND D.G. - MULLER S.A., *Androgenetic Alopecia (Common Baldness),* in *Cutis*, 1991, vol. 47 (3), p. 173; SASSON M. - SHUPACK J.L. - STILLER M.J., *Status of Medical Treatment for Androgenetic Alopecia*, in *International Journal of Dermatology,* 1993, vol. 32 (10), p. 701; SHEEN M., *Fighting Hair Loss,* USA Library Publishing, 1993-1996; OLSEN E.A. (ED.), *Disorders of Hair Growth,* New York, 1994; AMERICAN ACADEMY OF DERMATOLOGY, *Guidelines of Care for Androgenetic Alopecia,* in *Journal of American Academy of Dermatology,* 1996, vol. 35 (3 pt. 1), p. 465; KAUFMAN K.D., *Androgen Metabolism as if Affects Hair Growth in Androgenetic Alopecia,* in *Dermatologic Clinics,* 1996, vol. 14 (4), p. 697; SAWAYA M.E., *Clinical Updates in Hair,* in *Dermatologic Clinics,* 1997, vol. 15 (1), p. 37.

### Disclosure of Invention

The described cosmetic method intends to prevent hair loss and to stimulate the natural growth of hair in bald areas by restoring a valid micro-circulation into the scalp (both at cutaneous and subcutaneous level), thanks to the injection of an oxygene-ozone mixture.

It is self-evident the difference in comparison to the above mentioned methods, both for the result obtained (the prevention of hair loss and the natural growth of hair in their natural seat) and for the technique utilised (injections of a mixture of oxygen and ozone).

The invention is based upon two considerations.

Firstly, hair loss follows the alteration of the micro-circulation of the scalp. Its ongoing atrophy causes the loss of functionality of the hair follicle, that gradually becomes unable to generate hair. Like any tissue that slowly gets atrophic, the follicle, no more supported by the micro-circulation (and thus by oxygen and nutritive substances) produces at first a fragile hair, easy to tear out of its root. The subsequent step involves the spontaneous shedding of hair and the impossibility of its regeneration.

Secondly, if the scalp, for any reason, comes to tightly adhere to the cranial theca, hair cannot regrow owing to the correlated "suffocation" of the follicles.

On the basis of these considerations, it arose the idea to inject the oxygen-ozone mixture, in order to stimulate a "forgotten" or weakened process of hair production by the follicle and to restablish a proper space between the bony arch and the scalp: the latter, newly trophic, gets separated from the bone and allows the follicles to "breath" in a better way.

Those effects comes from the properties of ozone, that, though used in homeopathic doses, has regenerative, cicatrive and stimulating capacities of vessels and nerves.

It must be taken into account that ozone (O₃), being an allotrope of oxygen, is very instable and thus has a very short hemilife, trasforming in about one second into the stable oxygen (O₂). The extreme transience of the substance and its very nature (it is nothing else than oxygen) avoid any systemic effects: therefore collateral or allergical effects are not possible. For the same reason no contra-indication is provided.

The hereby described method is natural and does not have intrinsic limits to the number of applications, thus being able to adapt to the real needs of the single treated person.

Oxygen-ozone therapy is widely used, especially in countries such as Germany, Canada, Italy, Switzerland, Russia, Cuba and Eastern Europe countries. In brief, it can be said that ozone application is an effective and safe way of treating infections, bums, dental and intestinal conditions, circulatory disorders, discal hernia. Besides, blood ozonation has proven to be effective in improving immunological, endocrine, neurological, metabolic and hormonal systems; the inhibition and destruction of some cancers; the inactivation and/or destruction of viral, bacterial, fungal and yeast infections.

Uses of ozone are many, however, and not confined to the medical field. So far, ozone has been used in :
- aesthetics: for the cure of cellulitis and teleangectasy;
- industry, for the decontamination of waters and for the neutralization of industrial waste gas containing sulphate.

The primary methods of ozone therapy are major autohaemotherapy, rectal insufflation, ozone bagging, injections, ozonated oil.

For references, see, *inter alia,* MATTASSI R., *Ozonoterapia*, Milano, 1985; BOCCI V., *Autohaemotherapy after Treatment of Blood with Ozone. A Reappraisal*, in *Journal of International Medical Research,* 1994, vol. 22 (3), p. 131; VIEBAHN R., *The Use of Ozone in Medicine,* Heidelberg, 1994; MADEJ P. - ANTOSZEWSKI Z. - MADEJ J.A., *Ozonotherapy*, in *Materia Medica Polona*, 1995, Vol. 27 (2), p. 53; BOCCI V., *Ozone as a Bioregulator. Pharmacology and Toxicology of Ozonetherapy Today*, in *Journal of Biological Regulators and Homeostatic Agents*, 1996, Vol. 10 (2-3), p. 31.

### Modes for Carrying Out the Invention

The method basicly consists of the injection of an oxygen-ozone mixture in the area of the head corresponding to the location of the scalp. The needle must be introduced into a point immediately over the bony arch (preferably in a bald area), and the mixture must be injected between the scalp and the bone.

The result must be an homogeneous propagation of the mixture in the area of the head corresponding to the location of the scalp.

Given that the scalp often adheres to the cranial theca, if the mixture does not spread spontaneously around the area of the head corresponding to the location of the scalp, two or more injections are made in different points of the cranium (either in bald and non-bald areas), so that the gas detaches the scalp from the theca.

It is necessary, besides, to make a soft hand-massage on the interested areas, in order to ease the spread of the mixture around the area of the head corresponding to the location of the scalp.

The needles can have a lenght between 3 (three) millimetres and 50 (fifty) millimetres, and a diameter between 30 (thirty) G and 18 (eighteen) G.

As for the oxygen-ozone mixture, the concentration can vary from 1 (one) microgram O₃/ml.O₂ to 40 (fourty) micrograms O₃/ml.O₂, while the quantity can vary from 5 (five) to 60 (sixty) cubic centimetres.

Said values vary depending on the extensions of baldness and the reactivity of the person treated: the intrinsic nature of the treatment makes impossible to define an "ideal" mode for carrying out the method, having to pay due attention to the circumstances of the single case.

The same is true for the quantity and frequency of the injections. It is obviuos that, in order to obtain satisfactory and lasting effects, a sequence of injections is necessary: a reasonable way to proceed consists of injections repeated twice a week for about five weeks and then once a week for following two months. Afterwards, maintenance injections are usually necessary, monthly or bimonthly.

The characteristics of the hereby described method are further pointed out by means of an implementation example, given as a mere illustration and not intented to be exhaustive.

On a male subject, 48-aged, with a baldness developed since twenty years and interesting 80% of the scalp, the treatment started with the injection - by means of a needle 2 cm long, with a 27 G diameter - of a quantity of 20 cc. of mixture O₂-O_{3,} with a concentration of 2 micrograms O₃/ml.O₂.

Given that the gas did not spontaneously spread (owing to the adhesion of the scalp to the cranial theca), two additional injections (with the same parameters) were made in different sites of the cranium.

After the injection, a soft hand-massage was made, in order to ease the spread of the gas on the whole area of the head corresponding to the location of the scalp.

Afterwards, other sessions were made, with the following frequency: for the first five weeks, twice a week; for the following two months, once a week.

On the eight session, the concentration was increased to 6 micrograms O₃/ml.O₂ and the quantity to 40 cc.

During each session, the above described massage was made.

After the fifth session, the hair loss stopped and the scalp started appearing more trophic. At each session, it was observed that new follicles had been born.

After about one month and a half since the treatment were started, the hair regrowth process was self-evident. In particular, the regrowth process were constant, so that after one year from the beginning, strong hair was present on about 90% of the cranial arch, with a 50% density.

The new hair was black (while the temporal regions showed several white hair) and resistant to traction.

### Industrial Applicability

The described method is industrially applicable, given that it can be fulfilled, in the field of Aesthetical Treatments, to an indefinite number of persons affected by baldness or hairloss. It is easy to reproduce, needing only a person skilled in injections and the availability of a modem ozone generators, capable to deliver oxygen-ozone mixtures in needed amounts.

## Claims

1. Cosmetic Process for promoting hair growth carried out by means of injections with needle and **characterized by** the injection, in one or more points of the scalp, of an oxygen-ozone mixture, whose concentration can vary from 1 (one) microgram O₃/ml.O₂ to 40 (fourty) micrograms O₃/ml.O₂, and whose quantity can vary from 5 (five) to 60 (sixty) cubic centimetres.

2. Cosmetic Process for promoting hair growth carried out by means of injections with needle and **characterized by** the injection, in one or more points of the scalp, of an oxygen-ozone mixture, whose concentration can vary from 1 (one) microgram O₃/ml.O₂ to 40 (fourty) micrograms O₃/ml.O₂, and whose quantity can vary from 5 (five) to 60 (sixty) cubic centimetres. The injections are followed by a soft hand-massage aimed at spreading the mixture around the area of the head corresponding to the location of the scalp.

3. Process according to claim no. 1 or claim no. 2, **characterized by** the execution of periodical injections.

4. Process according to claim no. 1 or claim no. 2, **characterized by** the execution of two weekly sessions for five weeks and of one weekly session for the following two months, and by the execution, afterwards, of periodical maintenance sessions.

## Patentansprüche

1. Kosmetischer Prozess wegen Beförderung des Haareswaches durch Spritzen mit Nageln charakterisiert von der Einspritzung eines oder mehreren Punkten des Kopfes, mit einer Oxigen - Ozone Mischung, deren Konzentration von 1 (Ein) Mikrogramme O3/ ml O2 bis 40 (vierzig) Mikrogrammen O3/ ml O2 und deren Menge von 5 (Fünf) bis 60 (sechzig) Kubikzentimetern ändern kann.

2. Kosmetischer Prozess wegen Beförderung des Haareswaches durch Spritzen mit Nageln charakterisiert von der Einspritzung eines oder mehreren Punkten des Kopfes, mit einer Oxigen - Ozone Mischung, deren Konzentration von 1 (Ein) Mikrogramme O3/ ml O2 bis 40 (vierzig) Mikrogrammen O3/ ml O2 und deren Menge von 5 (Fünf) bis 60 (sechzig) Kubikzentimetern ändern kann. Die Einspritzungen werden von einer leichten Handmassierung gefolgt, womit die Verbreitung der Mischung über die betreffende Kopffläche erhalten wird.

3. Prozess betreffend Patentansprüche N° 1 oder N° 2 karakterisiert von der Aufführung periodischer Einspritzungen.

4. Prozess betreffend Patentansprüche N° 1 oder N° 2 karakterisiert von der Aufführung von 2 wöchentlichen Sitzungen während 5 Wochen und eine Sitzung pro Woche während der nächsten zwei Monaten. Hierauf folgt die Einführung von periodischen Unterhaltungssessionen.

## Revendications

1. Procès cosmétique pour la promotion de la pousse des cheveux par l'emploi d'aiguilles **caractérise par** l'injection, dans un ou plusieurs points du scalp, d'un mélange d'oxygène - ozone, dont la concentration peut varier de 1 (un) micro gramme O3/ml O2 à 40 (quarante) micro gramme O3/ ml O2 et dont la quantité peut varier de 5 (cinq) a 60 (soissante) centimètre cubiques.

2. Procès cosmétique pour la promotion de la pousse des cheveux par l'emploi d'aiguilles **caractérise par** l'injection, dans un ou plusieurs points du scalp, d'un mélange d'oxygène - ozone, dont la concentration peut varier de 1 (un) micro gramme O3/ml O2 à 40 (quarante) micro gramme O3/ ml O2 et dont la quantité peut varier de 5 (cinq) a 60 (soissante) centimètre cubiques. Les injection sont suivies d'un massage délicat manuel qui sert a répandre la mixture tout autour de la part de la tête correspondante a l'endroit du scalp.

3. Procès concernant la revendication N° 1 ou la revendication N° 2 **caractérisées par** l'exécution de injections périodique.

4. Procès concernant la revendication N° 1 ou la revendication N° 2 **caractérisées par** l'exécution de 2 sessions par semaine pour la durée de 5 semaines et de 1 session par semaine pendant les deux mois suivants et **caractérisées par** l'exécution, successivement, de sessions périodiques de maintien.
